# EUROPEAN PATENT APPLICATION

(11) **EP 1 676 585 A1**
(43) Date of publication of application: **05.07.2006**
(21) Application number: 06006010.0
(22) Date of filing: 17.05.2001
(51) Int. Cl.: A61K 36/02

(54) **Pepsin inhibition by alginates**

(30) Priority: 19.05.2000 GB 0012094
(62) Divisional of application: 01931845.0
(71) Applicant: Reckitt Benckiser Healthcare (UK) Limited, Slough, Berkshire SL1 3UH (GB)
(72) Inventor: Dettmar, Peter, Patrington East Yorkshire HU12 OPE (GB); Hampson, Frank Chadwick, Hedon East Yorkshire HU12 8HU (GB); Sunderland, Andrew Michael, Hull HU8 8DU (GB); Pearson, Jeffrey Peter, Waterhouses Durham DH7 9HE (GB)
(74) Representative: Dickson, Elizabeth Anne

(57) **Abstract**

Certain alginates exhibit a superior ability to inhibit the proteolytic activity of pepsin and/or gastric juice. In particular, alginates having a molecular weight between 40,000 and 350,000 exhibit a superior inhibition of the proteolytic activity of pepsin and gastric juice by over 50% when measured using an N-terminal assay.

## Description

The present invention relates to the use of alginates to inhibit enzymes. In particular this invention relates to the use of certain types of alginate to inhibit proteolytic enzymes.

The reflux of gastric contents into the oesophagus may give rise to damage to the squamous epithelium of the oesophagus and may predispose the oesophageal mucosa to Barrett's oesophagus and oesophageal carcinoma. The main aggressors of gastric refluxate are pepsin and hydrochloric acid. The acid can be rapidly neutralised with sodium bicarbonate, but the pepsin can remain active at pH's up to 5 and is only irreversibly inhibited at pH's of above 7. Pepsin is a proteolytic enzyme which catalyses the splitting of peptide linkages between certain amino acids. Consequently pepsin may have a continued damaging effect on the oesophagus.

One approach to the problem of reflux oesophagitis has been to administer a preparation which on contact with gastric acid generates a carbonated gelatinous foam or raft which floats on the stomach contents. When reflux occurs it is this raft which precedes the stomach contents into the oesophagus, thus protecting the mucosa from further irritation. Known preparations of this type include solid preparations in the form of powder or tablets containing alginic acid, sodium bicarbonate and antacid materials or liquid preparations containing sodium alginate, sodium bicarbonate and calcium carbonate marketed under the name GAVISCON (TM Reckitt & Colman Products Ltd). In our British Patent No. 1524740 we describe such liquid preparations.

In addition to physically preventing damage to the oesophagus, a need exists to inhibit the activity of the pepsin and/or gastric juice to thereby minimise the damage to the oesophagus.

We have now found that certain alginates inhibit pepsin and/or gastric juice activity better than other alginates.

Accordingly, the present invention provides the use of an alginate having a molecular weight of less than 400,000 in the manufacture of a pharmaceutical composition for the inhibition of the proteolytic activity of pepsin and/or gastric juice in a mammal.

Alginate is a mixture of polyuronic acids composed of residues of D-mannuronic and L-guluronic acids and may be obtained from known algae belonging to the order *Phaeophycae.* One of the most useful properties of alginates is their ability to form viscous solutions at low concentrations.

The term 'alginate' is intended to encompass alginic acids, salts of alginic acids (alginate salts), derivatives of alginic acid, for example, esters such as propylene glycol and mixtures thereof.

References to molecular weight are to weight average molecular weight.

The alginate is preferably a monovalent salt of alginic acid, for example, the sodium, potassium or ammonium salt, most preferably the sodium salt. It will be understood that the sodium alginates used in the present invention may contain minor amounts of other alginate salts for example calcium or potassium, e.g. up to about 10% by weight. Such alginates may be supplied by FMC Biopolymer AS, for example, Protanal H120L and Protanal LF120.

Average characteristics of some of the sodium alginates supplied by FMC Biopolymer AS are set out in Table 1 below.

**Table 1: Average characteristics of sodium alginates supplied by FMC Biopolymer AS**

| Source | Grade | Viscosity (1% solution) mPa.s | Molecular Weight | Guluronate Fraction Fg | G-block Length Ng>1 |
|---|---|---|---|---|---|
| *Laminaria hyperborea* | LFR 5/60 | 6 | 40,000 | 0.64 | 15.7 |
| *Laminaria hyperborea* | SF120 | 110 | 225,000 | 0.69 | 14.5 |
| *Laminaria hyperborea* | SF/LF | 410 | 315,000 | 0.63 | 20.3 |
| *Laminaria hyperborea* | SF200 | 990 | 380,000 | 0.69 | 20.3 |
| *Lessonia nigrescens* | LF10L | 9.3 | 75,000 | 0.45 | 6.0 |
| *Lessonia nigrescens* | LF120 | 121 | 245,000 | 0.44 | 6.9 |
| *Lessonia nigrescens* | SF60 | 368 | 320,000 | 0.44 | 7.4 |
| *Lessonia nigrescens* | H120L | 950 | 350,000 | 0.46 | 7.0 |

Preferably the alginate has a molecular weight less than 380,000, more preferably less than 365,000 and most preferably less than 350,000.

The alginate preferably has a molecular weight more than 30,000. Preferably, the alginate has a molecular weight more than 40,000, more preferably more than 60,000, most preferably more than 75,000.

It will be appreciated that any of the limitations of the alginate hereinbefore described may be combined in any combination and all combinations of the limitations are hereby expressly disclosed.

In a preferred embodiment of the present invention the alginates are selected from Protanal SF120, Protanal SF/LF, Protanal LF10L, Protanal LF120 and Protanal SF60.

The pepsin may be any pepsin, for example, porcine, equine, murine, ovine, bovine or human pepsin. Preferably the pepsin is human pepsin.

Proteolytic activity is defined as the breakdown of biological materials into simpler substances, such as proteins into smaller proteins and/or amino acids.

Gastric juice is defined as comprising water, hydrochloric acid (HCl), one or more salts including NaCl, KCl, CaCl₂, Ca₃(PO₄)₂, FePO₄ and Mg₃(PO₄)₂ and enzymes including pepsin. Gastric juice may be found in the stomach or gut of any mammal, including that of a human.

Preferably a 1% part by weight aqueous solution of the alginate has a viscosity of less than 600 mPa.s when measured on a Brookfield RVT viscometer using spindle number 3 at 20 r.p.m. at 20 degrees Celsius.

More preferably the alginate has a viscosity less than 550 mPa.s, most preferably less than 500 mPa.s.

The present invention is based on the surprising discovery that a selection of alginates exhibit a superior ability to inhibit the proteolytic activity of pepsin and/or gastric juice. In particular, alginates having a molecular weight from 40,000 to 350,000 exhibit a superior inhibition of the proteolytic activity of pepsin and gastric juice by over 50% when measured using an N-terminal assay.

Accordingly, the invention further provides the use of alginate to inhibit the proteolytic activity of pepsin and/or gastric juice wherein the alginate has a molecular weight less than 400,000.

According to a further embodiment of the present invention, there is provided the use of alginate in the manufacture of a pharmaceutical composition for the protection of the oesophagus from pepsin induced disease or damage.

According to a further embodiment of the present invention, there is provided the use of a sodium alginate from the source *Laminaria* having a guluronate block length of above 14.0 or a sodium alginate from the source *Lessonia* having a guluronate block length of at least 7.0, preferably at least 6.9, in the manufacture of a medicament for the protection of the oesophagus from pepsin induced disease or damage.

In particular, the use may be for the treatment or prevention of Barrett's oesophagus, oesophageal carcinoma, or pepsin induced gastric reflux damage.

The sodium alginates which are used in this aspect to the present invention are from the source *Laminaria* and have a guluronate block length of above 14.0. Preferred sodium alginates from the source *Laminaria* for use in the present invention are Grades LFR 5/60, SF 120, SF/LF and SF 200 available from FMC Biopolymer A/S, with grade SF 200 being most preferred. Further characteristics of examples of these sodium alginate grades are given in Table 1.

The sodium alginates from the source Laminaria used in this aspect to the present invention preferably have a guluronate fraction of at least 0.64, preferably above 0.68.

Preferred sodium alginates from the source *Lessonia* for use in the present invention are grades SF 60, HI20L and LF120L. Further characteristics of examples of these sodium alginate grades are given in Table 1.

The preferred sodium alginates from the source *Lessonia* preferably have a guluronate fraction of at least 0.44.

The present invention also includes within its scope a method for the protection of the oesophagus from pepsin induced disease or damage, which method comprises the administration to a patient of an orally effective amount of alginate having a molecular weight of less than 400,000.

The present invention also includes within its scope a method for the protection of the oesophagus from pepsin induced disease or damage, which method comprises the administration to a patient of an orally effective amount of sodium alginate from the source *Laminaria* having a guluronate block length of above 14.0 or a sodium alginate from the source *Lessonia* having a guluronate block length of at least 7.0, preferably at least 6.9.

The sodium alginate which is used in the present invention may be administered as a pharmaceutical composition which is formulated in a manner such that it is palatable and reacts with gastric acid to form a raft on the contents of the stomach.

The pharmaceutical compositions used in the present invention may be presented in the form of dry powders which can be admixed with water. It will readily be appreciated that the amount of water with which the dry powder is admixed should be so chosen that a palatable liquid preparation is obtained. However, in a preferred embodiment of the present invention the compositions are presented in liquid form.

In the pharmaceutical preparations in liquid form the sodium alginate is preferably contained in an amount of from 0.1 to 12.0% weight/volume, preferably 4.0 to 11.0% weight/volume. The liquid formulations may be provided in bulk or may be packaged in individual sachets in a unit dosage form.

The compositions used in the present invention preferably also comprise a suspending agent. Suitable suspending agents include carrageenan, hypromellose, tragacanth, pectin, pre-gelatinised potato starch, sodium starch glycolate, carbomer, xanthan gum or mixtures thereof. Carbomer is a synthetic high molecular weight polymer of acrylic acid cross linked with either allyl esters of sucrose or pentaerythritol. Suitable commercially available grades of carbomer include Carbopol 934P or Carbopol 974 (BF Goodrich).

For use in liquid products, carbomers must be neutralised after being pre-dispersed in water. The preferred neutralising agent is sodium hydroxide. The concentration of carbomer is given as the total amount of material used before neutralisation.

The choice of suspending agent and its concentration will depend upon the amount and grade of sodium alginate used in the compositions and upon the amount and type of extra insoluble ingredients used. Preferably the suspending agent is a carbomer. The preferred concentration of suspending agent is 0.1 to 1% w/v, most preferably 0.1 to 0.5% w/v.

The compositions of the present invention preferably further comprise a source of divalent or trivalent metal ions to strengthen the raft formed in the stomach. These metal ions preferably become available when the compositions reach the stomach but must not be available before then or the compositions will gel too early. Suitable metal ions are aluminium and, preferably, calcium ions. Most preferably the compositions comprise calcium carbonate.

The compositions used in the present invention therefore preferably further comprise from 0.1 to 5% w/v calcium ions, most preferably 0.5 to 3% w/v calcium carbonate.

The compositions of the present invention may further comprise preservatives to prevent contamination and subsequent deterioration by micro-organisms. Examples of suitable preservatives are methyl, ethyl, propyl and butyl para-hydroxybenzoates and their salts, which are preferably used in combination e.g. methyl and propyl or ethyl and butyl.

Preferred concentrations for the preservatives are 0.01 to 0.5% w/v.

The compositions of the present invention may also include one or more of the following ingredients, colouring, sweetening, flavouring or pH adjusting ingredients.

Where the compositions of the present invention are intended for use as sustained releasing compositions they will also contain active ingredients suitable for sustained administration in the stomach.

Where the compositions of the present invention are intended for use as targeted delivery compositions they will also contain active ingredients suitable for specific delivery to the stomach, for example local antimicrobial agents.

The present invention also includes within its scope a pharmaceutical composition including at least two, preferably at least three different grades of alginate, at least one of which has a molecular weight of less than 400,000, preferably from 40,000 to 350,000, more preferably from 100,000 to 300,000, most preferably from 200,000 to 255,000.

Preferably at least one alginate has a mannuronic acid to guluronic acid MG ratio of not more than 0.6:1, for example Protanal SF120, Protanal SF/LF40 and Protanal SF200.

Alternatively, or in conjunction with the above, preferably at least one alginate has a MG ratio of at least 1, for example Protanal LF120L, Protanal SF60L and Protanal H120L.

As such, the composition according to this aspect of the present invention provides a composition having pepsin inhibition activity as well as, very valuably, strong raft properties where the MG ratio is not more than 0.6:1 and/or superior mucoadhesive properties where the MG ratio is at least 1.

In this respect, the applicant refers to International Patent Publication No. WO98/48814, the contents of which are incorporated herein by reference.

The compositions of the invention may be prepared by any conventional manufacturing process for compositions of this type.

In the preferred embodiments of the invention, all references to % weight are to % weight per volume.

Any feature of any aspect of any invention or embodiment described herein may be combined with any feature of any aspect of any other invention or embodiment described herein.

The present invention will be further described with reference to the following Examples and Figures 1 to 4 in which:
Figure 1 shows the percentage inhibition of 2.0µg Porcine Pepsin A by 0.50mg of different molecular weight sodium alginates;
Figure 2 shows the percentage inhibition of 4.0µg Porcine Pepsin A by 0.50mg of different molecular weight sodium alginates;
Figure 3 shows the relationship between viscosity (1% part by weight solution) mPa.s and the percentage inhibition of 2.0µg Porcine Pepsin A by sodium alginate of different molecular weights; and
Figure 4 shows the relationship between viscosity (1% part by weight solution) mPa.s and the percentage inhibition of 4.0µg Porcine Pepsin A by sodium alginate of different molecular weights.

### METHOD OF ASSESSING PROTEOLYTIC ACTIVITY

A sensitive and accurate method for estimating proteolytic activity is by measuring trinitro-phenylated derivatives of new N-terminal groups which form on peptide bond hydrolysis. The sensitivity of the assay is improved by using succinyl albumin as the protein substrate in which pre-existing amino groups on the albumin protein substrate are blocked. The method can measure pepsin activity in samples of gastric juice.

### PREPARATION OF SOLUTIONS

### Succinyl Albumin Substrate

20g of bovine serum albumin (ICN, Cedarwood, Basingstoke)) were dissolved in 200ml of phosphate buffer pH 7.5. 2.8g of succinic anhydride were then added slowly, while maintaining the pH at 7.5 by the addition of drop wise 2M sodium hydroxide (NaOH). The resulting solution was dialysed exhaustively against deionised water at 4°C, freeze-dried and stored at -18°C. When required an 8.0mg/ml solution, in 0.01M hydrochloric acid (HCl), was made and drop wise 1M HCl added until the solution went clear, and then adjusted to pH 2.2 with 1M NaOH.

### Alginate Preparation

A fresh 5mg/ml solution of the required alginate (FMC Biopolymer, Drammen, Norway) was made up with deionised water and dilutions made thereof to produce 2.5mg/ml, 1.0mg/ml, 0.5mg/ml, 0.1mg/ml, and 0.05mg/ml solutions.

### Porcine Pepsin A Preparation

A 0.04mg/ml solution of Porcine Pepsin A (SIGMA Chemicals, Poole, Dorset), in 0.01M hydrochloric acid (HCl) (BDH), was freshly made prior to each assay.

### TNBS Preparation

1.5ml of 5% 2,4,6-trinitrobenzenesulphonic acid (TNBS), 1M in water, was mixed with 20mg of activated charcoal (BDH) and left at room temperature for 10 minutes before being centrifuged at 5000rpm for 10 minutes. The supernatant was then filtered through a 0.2µm syringe filter. This procedure removed some of the active compound of TNBS and any particles of charcoal, to optimise absorbance readings. 1.1ml of the resulting solution was then diluted 1:100 using deionised water.

### N-Terminal Assay Method

Pepsin activity was quantified using the N-terminal assay (Lin *et al* 1969, Hutton *et al* 1986 and 1990). It is sensitive to 0.1µl of pepsin and uses succinyl albumin substrate, and Porcine Pepsin A as standard. In this colourimetric assay N-terminal groups were triphenylated followed by the production of a complex compound of yellow colour. Standard curves were prepared using 0-200µl of pepsin solution made up to 200µl using 0.01M HCl. Test solutions were made up of 0, 50 and 100µl of pepsin solution made up to 100µl using 0.01M HCl, and to this 100µl of the required concentration of test alginate was added. All points were done in quintuplet. 500µl of succinyl albumin solution was added to each tube, whirly mixed, and incubated for 30 minutes at 37°C. Pepsin activity was arrested by the addition of 500µl of 4% (w/v) of freshly made sodium bicarbonate (NaHCO₃) and the tubes whirly mixed. Colour development of the newly formed N-terminals was done by the addition of 500µl of TNBS solution, and incubation at 50°C for 10 minutes. 500µl of 10% (w/v) sodium dodecyl sulphate (SDS) (BDH) was added to each tube to prevent protein precipitation followed by the addition of 250µl of 1.0M HCl to stop further colour development. The tubes were then left for 1 hour to settle at room temperature. The absorbance was measured at 340nm against deionised water and the percentage inhibition of pepsin activity estimated by comparison with the standard curve at 50µl (2.0µg) and 100µl (4.0µg) of pepsin.

### EXAMPLE 1

Alginates were diluted to produce 0.05mg/ml, 0.10mg/ml, 0.50mg/ml, 1.00mg/ml, 2.50mg/ml and 5.00mg/ml solutions, of which 100µl was used in the N-terminal assay. The alginate solutions were assayed with 50µl of a 0.04mg/ml solution of Porcine Pepsin A and any reduction in pepsin activity calculated against a pepsin standard curve.

The results are set out in Table 2 below.

**Table 2: Percent inhibition of 2.0 µg Porcine Pepsin A**

| Alginate | LFR560 | H120L | SF200 | SF60 | LF10L | LF120L | SF/LF | SF120 |
|---|---|---|---|---|---|---|---|---|
| 0.005mg | 23.18 | 15.05 | -2.73 | 18.70 | 16.47 | 7.11 | 14.35 | 16.43 |
| 0.01mg | 18.61 | 16.16 | -3.47 | 22.99 | 12.13 | 8.79 | 19.57 | 12.54 |
| 0.05mg | 20.49 | 22.36 | 5.20 | 25.69 | 5.73 | 19.12 | 15.19 | 8.04 |
| 0.10mg | 16.51 | 30.66 | 13.27 | 31.11 | 12.93 | 27.97 | 20.62 | 13.95 |
| 0.25mg | 39.96 | 44.54 | 31.07 | 57.18 | 39.27 | 51.98 | 47.68 | 41.18 |
| 0.50mg | 46.46 | 51.29 | 37.00 | 64.61 | 59.20 | 70.36 | 57.49 | 66.26 |

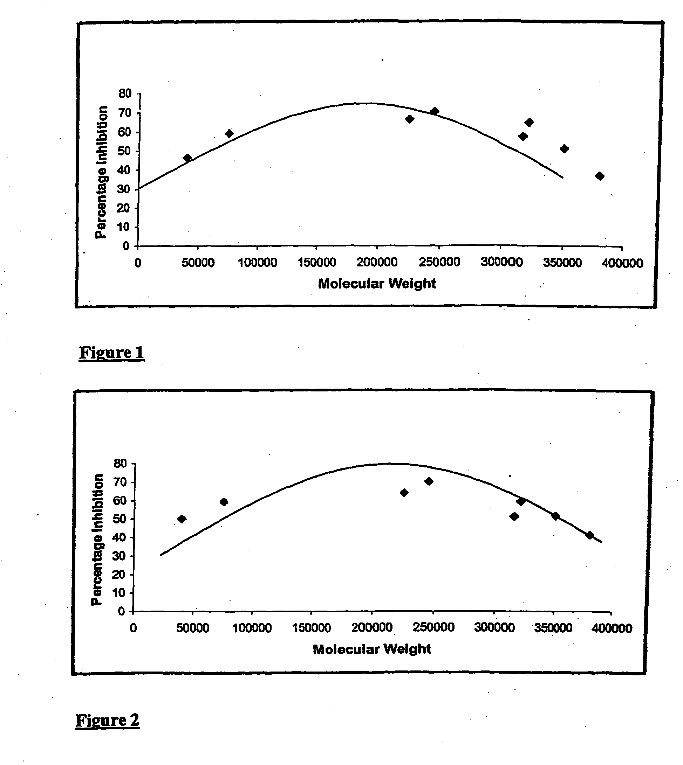

The results of the 0.50mg assay are further illustrated in Figure 1 which clearly shows the inverted U shaped curve illustrating the trend in the ability of the alginate to inhibit pepsin as a function of its molecular weight.

The same results, plotted as a function of viscosity rather than molecular weight, are shown in Figure 2.

### EXAMPLE 2

Alginates were diluted to produce 0.05mg/ml, 0.10mg/ml, 0.50rng/ml, 1.00mg/ml, 2.50mg/ml and 5.00mg/ml solutions, of which 100µl was used in the N-terminal assay. The alginate solutions were assayed with 100µl of a 0.04mg/ml solution of Porcine Pepsin A and any reduction in pepsin activity calculated against a pepsin standard curve.

The results are set out in Table 3 below.

**Table 3: Percent inhibition of 4.0 µg Porcine Pepsin A**

| Alginate | LFR560 | H120L | SF200 | SF60 | LF10L | LF120L | SF/LF | SF120 |
|---|---|---|---|---|---|---|---|---|
| 0.005mg | 15.00 | 12.50 | 2.92 | 15.04 | 7.85 | 9.89 | 4.75 | 7.77 |
| 0.01mg | 13.67 | 14.34 | 2.27 | 13.63 | 7.06 | 9.50 | 5.36 | 9.11 |
| 0.05mg | 15.50 | 18.83 | 5.04 | 19.68 | 10.40 | 13.93 | 12.70 | 10.75 |
| 0.10mg | 16.35 | 26.76 | 13.64 | 27.54 | 15.66 | 22.25 | 21.52 | 16.21 |
| 0.25mg | 38.50 | 43.65 | 34.05 | 49.17 | 38.81 | 51.65 | 43.03 | 42.31 |
| 0.50mg | 50.01 | 51.72 | 41.58 | 59.32 | 59.11 | 70.09 | 51.39 | 64.09 |

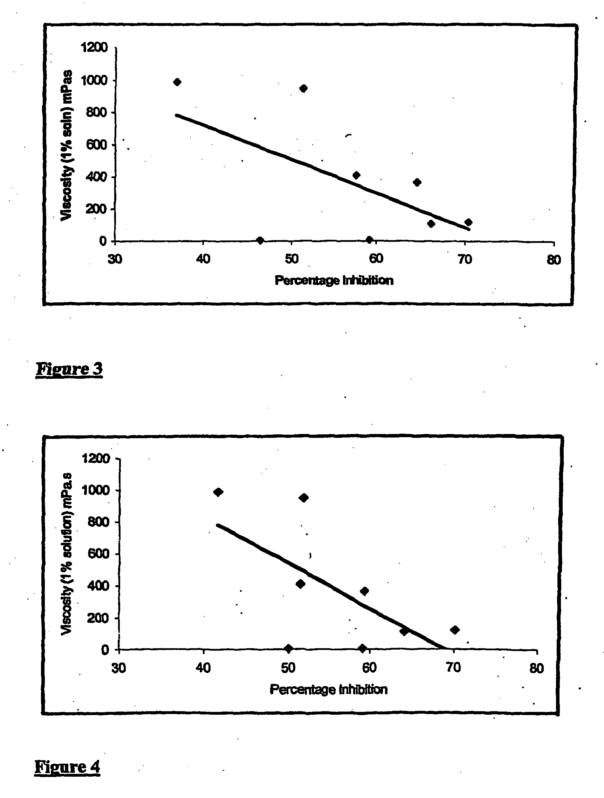

The results of the 0.50mg assay are further illustrated in Figure 3 which clearly shows the inverted U shaped curve illustrating the trend in the ability of the alginate to inhibit pepsin as a function of its molecular weight.

The same results, plotted as a function of viscosity rather than molecular weight, are shown in Figure 4.

### EXAMPLE 3

Samples of 200µl human gastric juice were assayed for pepsin activity against a Porcine Pepsin A standard curve, and then assayed with alginates LF120L, H120L and SF200. These alginates were tested due to their range of Porcine Pepsin A inhibition.

The results of the assay are set out in Table 4 below.

**Table 4: Percentage inhibition of two volumes gastric juice by alginates H120L, SF200 and LF120L.**

| **Alginate** | **H120L** | | **SF200** | | **LF120L** | |
|---|---|---|---|---|---|---|
| **µl Gastric Juice** | **50µl** | **100µl** | **50µl** | **100µl** | **50µl** | **100µl** |
| **0.005mg** | 2.376 | -0.760 | 1.709 | -16.253 | 7.051 | -20.090 |
| **0.010mg** | 4.752 | -0.285 | -11.966 | -19.639 | 0.855 | -17.720 |
| **0.050mg** | 0.864 | 3.989 | 3.632 | -9.029 | 5.128 | -3.499 |
| **0.100mg** | 12.959 | 16.904 | 4.487 | 1.242 | 9.615 | 5.079 |
| **0.250mg** | 44.924 | 40.171 | 30.769 | 24.944 | 54.487 | 42.099 |
| **0.500mg** | 42.333 | 48.338 | 29.700 | 39.165 | 56.197 | 52.930 |

### EXAMPLE 4

A composition containing

| | |
|---|---|
| Sodium alginate LFR 5/60 | 100g |
| Sodium bicarbonate | 26g |
| Calcium carbonate | 32g |
| Monopotassium phosphate | 0.6g |
| Dipotassium phosphate | 5.4g |
| Ethyl parahydroxybenzoate | 2g |
| Butyl parahydroxybenzoate | 0.2g |
| Sodium saccharin | 1g |
| Flavour | 0.7g |
| Deionised water | to 1 litre |

is made up as follows
1. 917ml of deionised water are dispensed into a mixing vessel and cooled to approximately 20°C.
2. The monopotassium phosphate and dipotassium phosphate are added and stirred until dissolved.
3. The preservatives, carbonates and sweetener are added to the mixture and stirred for 5 minutes.
4. The alginate is added with stirring over a period of 3 minutes.
5. The mixture is stirred for 30 minutes (the flavour being added after 10 minutes).
6. The temperature is controlled during manufacture to 22°C (plus or minus 5°C).

### EXAMPLES 5 to 9

The following examples are all produced according to the method of Example 8 using the amounts of components as set out in the table below.

| | EXAMPLE | | | | |
|---|---|---|---|---|---|
| | 5 | 6 | 7 | 8 | 9 |
| Sodium alginate LF120 | 20g | 75g | 20g | 75g | 25g |
| Sodium alginate LFR5/60 | 70g | 25g | 70g | 25g | 70g |
| Sodium alginate SF120 | log | - | log | - | 5g |
| Sodium Bicarbonate | 26g | 26g | 26g | 16g | 20g |
| Calcium carbonate | 32g | 16g | 60g | 32g | 32g |
| Potassium phosphate | 0.6g | 0.6g | 0.6g | 0.6g | 0.6g |
| Dipotassium phosphate | 5.4g | 5.4g | 5.4g | 5.4g | 5.4g |
| Ethyl parahydroxybenzoate | 2.0g | 2.0g | 2.0g | 2.0g | 2.0g |
| Butyl parahydroxybenzoate | 0.2g | 0.2g | 0.2g | 0.2g | 0.2g |
| Sodium saccharin | 1.0g | 1.0g | 1.0g | 1.0g | 1.0g |
| Flavour | 0.7g | 0.7g | 0.7g | 0.7g | 0.7g |
| Deionised water to | 11tr | 11tr | 11tr | 11tr | 11tr |

## Claims

1. Use of an alginate, preferably a monovalent salt of alginic acid, more preferably the sodium salt of alginic acid having a molecular weight of more than 30,000 and less than 380,000 preferably from 40,000 to 350,000 in the manufacture of a pharmaceutical composition for the inhibition of the proteolytic activity of pepsin and/or gastric juice in a mammal.

2. Use as claimed in claim 1 wherein the alginate has a molecular weight less than 365,000 and preferably more than 40,000.

3. Use as claimed in any one of claims 1 and 2 wherein the pepsin is porcine, equine, murine, ovine, bovine or human pepsin.

4. Use as claimed in any one of claims 1 to 3 wherein a 1% part by weight aqueous solution of the alginate has a viscosity of less than 600 mPa.s preferably less than 550 mPa.s when measured on a Brookfield RVT viscometer using spindle number 3 at 20 r.p.m. at 20 degrees Celsius.

5. Use of alginate to inhibit the proteolytic activity of pepsin and/or gastric juice wherein the alginate has a molecular weight of more than 30,000 and less than 380,000.

6. Use of alginate in the manufacture of a pharmaceutical composition for the protection of the oesophagus from pepsin induced disease or damage.

7. The use as claimed in claim 6 for the treatment or prevention of Barrett's oesophagus or oesophageal carcinoma.

8. The use as claimed in any one of the preceding claims wherein the sodium alginate is provided in the form of a liquid pharmaceutical preparation preferably packaged in individual sachets in a unit dosage form.

9. A pharmaceutical composition including at least three different grades of alginate, at least one of which has a molecular weight of less than 400,000, preferably from 40,000 to 350,000, more preferably from 100,000 to 300,000, most preferably from 200,000 to 255,000 and at least one of which alginates has a mannuronic acid to guluronic acid MG ratio of not more than 0.6:1 and at least one of which alginates has a MG ratio of at least 1.
